# EUROPÄISCHE PATENTSCHRIFT

(11) **EP 2 430 994 B1**
(45) Veröffentlichungstag und Bekanntmachung des Hinweises auf die Patenterteilung: **05.07.2017**
(21) Anmeldenummer: 11181730.0
(22) Anmeldetag: 19.09.2011
(51) Int. Cl.: A61B 17/70

(54) **Wirbelsäulenstabilisierungssystem, Verbindungselement für ein Wirbelsäulenstabilisierungssystem und Verfahren zum Herstellen eines solchen Verbindungselements**
Spinal column stabilization system, connecting element for a spinal column stabilzation system and method of manufacturing such a connecting element
Système de stabilisation de la colonne vertébrale, élément de liaison pour un système de stabilisation de la colonne vertébrale et procédé de fabrication d'un tel élément de liaison

(30) Priorität: 20.09.2010 DE 102010037665; 21.10.2010 DE 102010060112
(43) Veröffentlichungstag der Anmeldung: 21.03.2012
(73) Patentinhaber: Aesculap AG, 78532 Tuttlingen (DE)
(72) Erfinder: Beger, Jens, 78532 Tuttlingen (DE); Hoefer, Fabian, 78532 Tuttlingen (DE); Hohl, Berthold, 78532 Tuttlingen (DE); Schmid, Stefan, 78570 Mühlheim (DE); Heine, Josef, 78532 Tuttlingen (DE)
(74) Vertreter: Hoeger, Stellrecht & Partner Patentanwälte mbB

(56) Entgegenhaltungen:
- WO-A1-2009/036156
- WO-A1-2010/099316
- FR-A1- 2 905 847
- US-A1- 2006 184 171
- US-A1- 2006 229 608
- US-A1- 2007 191 832
- US-A1- 2008 234 736

## Beschreibung

Die vorliegende Erfindung betrifft ein Verbindungselement für ein Wirbelsäulenstabilisierungssystem mit einem ersten Befestigungsabschnitt zum Festlegen an einer ersten Knochenbefestigungseinrichtung, einem zweiten Befestigungsabschnitt zum Festlegen an einer zweiten Knochenbefestigungseinrichtung und einem zwischen dem ersten und dem zweiten Befestigungsabschnitt angeordneten oder ausgebildeten, mindestens teilweise flexiblen Zwischenabschnitt, welcher Zwischenabschnitt in Form eines streifenförmigen, gewundenen Blattfederelementes ausgebildet ist und mindestens eine in einer Richtung quer zu einer vom Zwischenabschnitt definierten Längsachse seitlich geöffnete Ausnehmung aufweist, wobei mindestens eine der beiden Blattfederelementoberflächen des Blattfederelements mindestens ein Steifigkeitsänderungselement umfasst.

Des Weiteren betrifft die vorliegende Erfindung ein Wirbelsäulenstabilisierungssystem umfassend mindestens eine erste Knochenbefestigungseinrichtung, mindestens eine zweite Knochenbefestigungseinrichtung und ein Verbindungselement, welches Verbindungselement einen ersten Befestigungsabschnitt zum Festlegen an der mindestens einen ersten Knochenbefestigungseinrichtung, einen zweiten Befestigungsabschnitt zum Festlegen an der mindestens einen zweiten Knochenbefestigungseinrichtung und einen zwischen dem ersten und dem zweiten Befestigungsabschnitt angeordneten oder ausgebildeten, mindestens teilweise flexiblen Zwischenabschnitt umfasst, welcher Zwischenabschnitt in Form eines streifenförmigen, gewundenen Blattfederelements ausgebildet ist und mindestens eine in einer Richtung quer zu einer vom Zwischenabschnitt definierten Längsachse seitlich geöffnete Ausnehmung aufweist, wobei mindestens eine der beiden Blattfederelementoberflächen des Blattfederelements mindestens ein Steifigkeitsänderungselement umfasst.

Ferner betrifft die vorliegende Erfindung ein Verfahren zum Herstellen eines Verbindungselements für ein Wirbelsäulenstabilisierungssystem mit einem ersten Befestigungsabschnitt zum Festlegen an einer ersten Knochenbefestigungseinrichtung, einem zweiten Befestigungsabschnitt zum Festlegen an einer zweiten Knochenbefestigungseinrichtung und einem zwischen dem ersten und dem zweiten Befestigungsabschnitt angeordneten oder ausgebildeten, mindestens teilweise flexiblen Zwischenabschnitt, welcher Zwischenabschnitt in Form eines streifenförmigen, gewundenen Blattfederelements ausgebildet ist und mindestens eine in einer Richtung quer zu einer vom Zwischenabschnitt definierten Längsachse seitlich geöffnete Ausnehmung aufweist, wobei auf mindestens einer der beiden Blattfederelementoberflächen des Blattfederelements mindestens ein Steifigkeitsänderungselement ausgebildet wird.

Verbindungselemente und Wirbelsäulenstabilisierungssysteme der eingangs beschriebenen Art sind beispielsweise aus der US 2008/0234736 A1, der US 2006/0184171 A1, der FR 2 905 847 A1 und der WO 2009/036156 A1 bekannt. Bei den bekannten Verbindungselementen hängt die gewünschte Steifigkeit derselben, insbesondere von deren Zwischenabschnitt, von den jeweiligen Fertigungstoleranzen ab. Ferner ist es bei den bekannten Verbindungselementen nicht möglich, die Steifigkeit des Zwischenabschnitts gezielt in kleinen Bereichen während des Fertigungsprozesses oder aber auch später einzustellen. Eine solche "Feinjustierung" wäre jedoch vorteilhaft. Des Weiteren sind dünnwandige, federnde Bauteile schwierig zu fertigen, wobei insbesondere eine hohe Oberflächenqualität mit spanenden Bearbeitungsverfahren nur schwer zu erreichen ist. Folge hiervon sind wieder unvermeidliche Fertigungstoleranzen. Zwar können Blattfederelemente beispielsweise gut mittels Drahterodierverfahren hergestellt werden, allerdings kann das Gefüge des erodierten Materials an der Oberfläche nachteilig verändert werden. Außerdem müssen in der Regel Rückstände des Erodierdrahtes durch chemische Verfahren wie zum Beispiel Beizen entfernt werden, was wiederum zu weniger definierten Dimensionen und Oberflächen führt. Darüber hinaus ermöglichen es die bekannten Verbindungselemente nicht, deren Steifigkeit gegebenenfalls auch intra-operativ zu verändern.

Weitere Verbindungselemente und Wirbelsäulenstabilisierungssysteme sind zudem aus der WO 2010/099316 A1, der US 2007/0191832 A1 sowie der US 2006/0229608 A1 bekannt.

Es ist daher eine Aufgabe der vorliegenden Erfindung, ein Verbindungselement für ein Wirbelsäulenstabilisierungssystem, ein Wirbelsäulenstabilisierungssystem und ein Verfahren der eingangs beschriebenen Art so zu verbessern, dass eine gewünschte Federsteifigkeit insbesondere des Zwischenabschnitts unabhängig von eventuellen Fertigungstoleranzen des Fertigungsprozesses möglichst genau eingehalten wird.

Diese Aufgabe wird bei einem Verbindungselement der eingangs beschriebenen Art erfindungsgemäß dadurch gelöst, dass sich das Steifigkeitsänderungselement in einer vom Blattfederelement definierten Längsrichtung erstreckt und dass das Blattfederelement unterschiedliche Dicken diesseits und jenseits des mindestens einen Steifigkeitsänderungselements aufweist.

Das mindestens eine Steifigkeitsänderungselement kann insbesondere genutzt werden, um eine aufgrund von Fertigungstoleranzen bei der Herstellung des Zwischenabschnitts vom Sollwert abweichende Federsteifigkeit des Zwischenabschnitts gezielt zu korrigieren. Dies kann insbesondere direkt bei der Herstellung erfolgen, also bei der Fertigung des Verbindungselements, oder gegebenenfalls auch zu einem späteren Zeitpunkt, zum Beispiel intra-operativ durch Operateur. Selbstverständlich können beide Blattfederelementoberflächen des Blattfederelements ein oder mehrere Steifigkeitsänderungselemente umfassen. Diese können insbesondere parallel zueinander oder aber auch beabstandet voneinander eine gemeinsame Längsrichtung definieren oder seitlich zueinander versetzt angeordnet oder ausgebildet sein. Vorteilhaft ist es, dass das Blattfederelement unterschiedliche Dicken diesseits und jenseits des mindestens einen Steifigkeitsänderungselements aufweist. Diese Ausgestaltung ermöglicht es, Anforderungen an die Fertigungstoleranzen bei der Herstellung des Zwischenabschnitts etwas herabzusetzen. Auf diese Weise vereinfacht sich die Herstellung des Verbindungselements, da sich auf die Steifigkeit des Zwischenabschnitts auswirkende Fertigungstoleranzen durch eine entsprechende Ausgestaltung des Steifigkeitsänderungselements gezielt korrigieren lassen. Insbesondere ist so auch eine Fertigung des Blattfederelements durch Fräsen mittels zwei in entgegengesetzte Richtungen weisenden Fräsern möglich, welche die Ausnehmung zunächst durch zwei durch eine Trennwand voneinander getrennte Vertiefungen ausbilden. Wandstärken des Blattfederelements können dabei insbesondere diesseits und jenseits der Trennwand, das heißt diesseits und jenseits des Steifigkeitsänderungselements, bedingt durch Fertigungstoleranzen variieren.

Besonders einfach und stabil herstellen lässt sich das Verbindungselement, wenn das mindestens eine Steifigkeitsänderungselement und das Blattfederelement einstückig ausgebildet sind.

Die Herstellung des Verbindungselements lässt sich weiter vereinfachen, wenn das mindestens eine Steifigkeitsänderungselement streifenförmig ausgebildet ist. Vorzugsweise erstreckt sich das Steifigkeitsänderungselement in einer vom Blattfederelement definierten Längsrichtung. Denkbar wäre es jedoch auch, dass mindestens eine Steifigkeitsänderungselement quer zu dieser Längsrichtung auszubilden.

Da sich Fertigungstoleranzen typischerweise auf das Blattfederelement insgesamt auswirken, ist es günstig, wenn sich das mindestens eine Steifigkeitsänderungselement längs der gesamten Länge des Blattfederelements erstreckt. Beispielsweise kann es in Form eines Längsstreifens vom einen Ende bis zum anderen Ende des Blattfederelements reichen, sich also zwischen den beiden Befestigungsabschnitten längs der gesamten Länge des Zwischenabschnitts erstrecken. Optional können beide Blattfederelementoberflächen ein solches Steifigkeitsänderungselement aufweisen.

Vorteilhaft ist es, wenn beide Blattfederelementoberflächen des Blattfederelements jeweils mindestens ein Steifigkeitsänderungselement umfassen. Diese können in Abhängigkeit von der jeweiligen Blattfederelementoberfläche identisch oder optional unterschiedlich ausgebildet sein.

Gemäß einer bevorzugten Ausführungsform der Erfindung kann vorgesehen sein, dass das mindestens eine Steifigkeitsänderungselement in Form eines von einer der beiden Blattfederelementoberflächen abstehenden Versteifungsvorsprungs ausgebildet ist. Ein solcher Versteifungsvorsprung lässt sich fertigungstechnisch besonders einfach herstellen, wenn die Ausnehmung des Blattfederelements spanend mittels Stirn- beziehungsweise Schaftfräsern von zwei entgegengesetzten Seiten präpariert und zunächst eine Trennwand, die die von beiden Seiten ausgebildeten Vertiefungen voneinander trennt, stehen gelassen wird. Eine solche Trennwand und ein gegebenenfalls nach teilweise Entfernung derselben noch verbleibender Versteifungsvorsprung können komplett belassen oder aber auch anschließend teilweise entfernt werden, wobei eine Steifigkeit des Blattfederelements in Abhängigkeit des verbleibenden Versteifungsvorsprungs, das heißt in Abhängigkeit von dessen Größe und/oder Erstreckung und/oder dessen Dicke, in gewünschter und gezielter Weise eingestellt oder gegebenenfalls nachjustiert werden kann. Durch den Versteifungsvorsprung lässt sich insbesondere eine aufgrund der Herstellung zu geringe Steifigkeit auf einfache Weise und hochpräzise erhöhen.

Um gegebenenfalls eine etwas erhöhte Steifigkeit des Zwischenabschnitts am Ende des Fertigungsprozesses etwas zu verringern, kann es vorteilhaft sein, wenn das mindestens eine Steifigkeitsänderungselement in Form einer sich von einer der beiden Blattfederelementoberflächen in das Blattfederelement hinein erstreckenden Versteifungsnut ausgebildet ist. Eine solche Versteifungsnut lässt sich auf einfache Weise beispielsweise mit einem T-Fräser herstellen. Ebenso wie der Versteifungsvorsprung kann sich eine Versteifungsnut in Längsrichtung des Blattfederelements erstrecken oder gegebenenfalls auch quer dazu. Es kann lediglich eine einzige Versteifungsnut vorgesehen sein oder auch mehrere, welche beabstandet hintereinander und/oder seitlich versetzt zueinander angeordnet oder ausgebildet sein können.

Vorteilhafterweise erstreckt sich das mindestens eine Steifigkeitsänderungselement zwischen zwei aufeinander zu weisenden Oberflächenabschnitten einer der beiden Blattfederelementoberflächen. Mit anderen Worten kann das Steifigkeitsänderungselement zum Beispiel im Bereich einer Ausnehmung ausgeordnet sein. Es kann insbesondere stegförmig oder in Form einer Art "Schwimmhaut" ausgebildet sein und so eine Windung des Blattfederelements gezielt in seiner Steifigkeit erhöhen.

Besonders vorteilhaft ist es, wenn das mindestens eine Steifigkeitsänderungselement im Bereich der mindestens einen Ausnehmung angeordnet ist. Insbesondere kann es so groß angeordnet und ausgebildet sein, dass es die Ausnehmung quer zur Längsachse des Verbindungselements vollständig verschließt. Allerdings kann eine Dicke des Versteifungselements nur einen Bruchteil der Breite des Zwischenabschnitts in diesem Bereich betragen. Insbesondere kann das Steifigkeitsänderungselement eine Dicke aufweisen, die kleiner als eine Dicke des Blattfederelements ist.

Besonders einfach lässt sich das Verbindungselement herstellen, wenn das Verbindungselement spiegelsymmetrisch oder im Wesentlichen spiegelsymmetrisch zu einer Spiegelebene ausgebildet ist. Im Wesentlichen spiegelsymmetrisch bedeutet insbesondere, dass aufgrund von Fertigungstoleranzen bei einer Herstellung des Zwischenabschnitts aus einem Vollmaterial keine durchgehende Blattfederelementoberfläche hergestellt werden kann, sondern dass diese mindestens einen einstufigen Absatz aufgrund leicht versetzt eingeführter Fräser oder anderer spanender Bearbeitungswerkzeuge aufweisen kann.

Die Stabilität sowie die Biegeeigenschaften des Verbindungselements lassen sich besonders einfach und hochpräzise einstellen, wenn die Spiegelebene eine vom Verbindungselement definierte Längsachse enthält.

Das Verbindungselement lässt sich insbesondere hervorragend als Ersatz eines geraden, stabförmigen Verbindungselements nutzen, wenn die Längsachse Längsachsen der Befestigungsabschnitte definiert.

Besonders einfach herstellen lässt sich das Verbindungselement sowie eine Steifigkeit desselben einstellen lässt sich, wenn das mindestens eine Steifigkeitsänderungselement spiegelsymmetrisch oder im Wesentlichen spiegelsymmetrisch zur Spiegelebene ausgebildet ist. Beispielsweise kann es wie oben beschrieben durch spezielle Fertigungsverfahren in Form einer Trennwand ausgebildet sein, welche spiegelsymmetrisch zur Spiegelebene ausgebildet ist.

Gemäß einer weiteren bevorzugten Ausführungsform der Erfindung kann vorgesehen sein, dass das Verbindungselement mindestens eine sich quer zu einer vom Verbindungselement definierten Längsachse erstreckende Durchbrechung aufweist, welche mindestens abschnittsweise vom mindestens einen Steifigkeitsänderungselement begrenzt ist. In Abhängigkeit der Größe der Durchbrechung kann eine durch das Steifigkeitsänderungselement bewirkte Steifigkeitsänderung des Blattfederelements gezielt angepasst werden. Insbesondere ist es denkbar, dass das Steifigkeitsänderungselement die Durchbrechung vollständig umgibt, insbesondere kann es allseitig an die Durchbrechung angrenzen.

Beispielsweise zur Ausbildung von Verbindungsstegen am Blattfederelement kann es vorteilhaft sein, wenn die Durchbrechung mindestens teilweise vom Blattfederelement begrenzt ist. Sie kann dann insbesondere auch teilweise vom Steifigkeitsänderungselement begrenzt werden.

Vorteilhaft ist es, wenn eine Dicke des mindestens einen Steifigkeitsänderungselements in einer Richtung quer oder im Wesentlichen quer zu einer vom Verbindungselement definierten Längsachse maximal etwa einer Dicke des Blattfederelements entspricht. Die Dicke des Steifigkeitsänderungselements kann insbesondere für eine Anpassung der Steifigkeit des Zwischenabschnitts, beispielsweise zum Ausgleich von Fertigungstoleranzen, dienen. Da die Steifigkeit in erster Linie bestimmt werden soll durch das Blattfederelement, ist es günstig, wenn die Dicke des Steifigkeitsänderungselements in der angegebenen Weise gewählt wird.

Vorzugsweise liegt die Dicke des mindestens einen Steifigkeitsänderungselements in einem Bereich von etwa 0,2 mm bis etwa 0,6 mm. Günstig ist es, wenn sie in einem Bereich von etwa 0,3 mm bis etwa 0,5 mm liegt.

Günstigerweise liegt die Dicke des Blattfederelements in einem Bereich von etwa 0,5 mm bis etwa 1,5 mm. Vorzugsweise liegt die Dicke des Blattfederelements in einem Bereich von etwa 0,5 mm bis etwa 0,9 mm. Vorzugsweise liegt sie in einem Bereich von etwa 0,6 mm bis etwa 0,8 mm. Die Dicke des Blattfederelements kann insbesondere variieren. Beispielsweise können gekrümmte Abschnitte dicker sein als geradlinige Abschnitte des Blattfederelements. Insbesondere kann die Dicke eines gekrümmten Blattfederabschnitts im Bereich von etwa 0,7 mm bis etwa 1,2 mm liegen. Beispielsweise kann sie einen Wert von 0,975 mm aufweisen. Insbesondere kann die Dicke eines geradlinigen Blattfederabschnitts im Bereich von etwa 0,5 mm bis etwa 0,9 mm liegen. Beispielsweise kann sie einen Wert von 0,775 mm aufweisen.

Gemäß einer weiteren bevorzugten Ausführungsform der Erfindung kann vorgesehen sein, dass mindestens eine der beiden Blattfederelementoberflächen mindestens zwei stufenförmig ausgebildete Absätze aufweist, so dass die mindestens eine der beiden Blattfederelementoberflächen mindestens zwei, vorzugsweise drei ebene, parallel zueinander verlaufende Blattfederelementoberflächenabschnitte aufweist. Derartig geformte Blattfederelementoberflächen können beispielsweise einen streifenförmigen Vorsprung, welcher auch als Rippe bezeichnet werden kann und zur Ausbildung eines Steifigkeitsänderungselements dient, oder aber eine Versteifungsnut, welche ebenfalls zwei Oberflächenabschnitte der Blattfeder voneinander trennt, aufweisen. Die stufenförmigen Absätze der Blattfederelementoberflächen können in Abhängigkeit von der Höhe der jeweiligen Absätze gezielt genutzt werden, um eine von einem Sollwert abweichende Steifigkeit des Zwischenabschnitts gezielt auf den Sollwert nachzujustieren.

Vorzugsweise ist die mindestens eine Ausnehmung in zwei voneinander entgegengesetzte Richtungen weisend seitlich geöffnet. Dies kann beispielsweise dadurch erreicht werden, dass mittels zweier Fräser in aufeinander zu weisende Richtungen die Ausnehmung präpariert wird derart, dass zwischen den beiden ausgebildeten Vertiefungen eine Trennwand verbleibt. Optional kann die Trennwand auch teilweise entfernt werden.

Zu einer Erhöhung der Stabilität des Verbindungselements ist es günstig, wenn es einstückig ausgebildet ist.

Oberflächen hoher Güte lassen sich insbesondere dann erreichen, wenn das Verbindungselement durch spanende Bearbeitung aus einem Vollmaterial hergestellt ist. Eine Dauerfestigkeit des Verbindungselements hängt insbesondere von einer Qualität der Oberflächen des Verbindungselements ab. Diese kann gegebenenfalls nach einer spanenden Bearbeitung durch eine anschließende Oberflächenbehandlung erhöht werden.

Günstig ist es, wenn das Verbindungselement aus einem metallischen Material oder aus einem Kunststoff hergestellt ist. Je nach erforderlicher Steifigkeit kann das eine oder das andere Material zur Herstellung des Verbindungselements gewählt werden. Insbesondere können durch eine Wahl des Materials praktisch beliebige Steifigkeiten eingestellt werden. Vorzugsweise liegen diese in einem Bereich von etwa 30 N/mm bis 150 N/mm.

Vorzugsweise ist das metallische Material Titan, eine Titanlegierung oder eine Kobalt-Chrom-Legierung oder enthält die genannten Materialien. Insbesondere handelt es sich dabei um körperverträgliche metallische Materialien.

Günstigerweise ist der Kunststoff Polyetheretherketon (PEEK) oder kohlefaserverstärktes Polyetheretherketon (PEEK) oder enthält die genannten Materialien. Insbesondere die genannten Materialien zeichnen sich durch eine hohe Körperverträglichkeit aus.

Die eingangs gestellte Aufgabe wird bei einem Wirbelsäulenstabilisierungssystem erfindungsgemäß dadurch gelöst, dass sich das Steifigkeitsänderungselement in einer vom Blattfederelement definierten Längsrichtung erstreckt und dass das Blattfederelement unterschiedliche Dicken diesseits und jenseits des mindestens einen Steifigkeitsänderungselements aufweist.

Ein derart verbessertes Verbindungselement des Wirbelsäulenstabilisierungssystems weist die bereits oben beschriebenen Vorteile auf und dient somit auch zu einer Verbesserung des Wirbelsäulenstabilisierungssystems insgesamt.

Ferner ist es günstig, wenn das Verbindungselement des Wirbelsäulenstabilisierungssystems in Form eines der oben beschriebenen, bevorzugten Ausführungsformen ausgebildet ist. Das Wirbelsäulenstabilisierungssystem weist dann insgesamt auch die Vorteile der verbesserten Verbindungselemente auf.

Die eingangs gestellte Aufgabe wird bei einem Verfahren der eingangs beschriebenen Art zum Herstellen eines Verbindungselements für ein Wirbelsäulenstabilisierungssystem erfindungsgemäß dadurch gelöst, dass das Steifigkeitsänderungselement in einer vom Blattfederelement definierten Längsrichtung erstreckend ausgebildet wird und dass das Blattfederelement mit unterschiedliche Dicken diesseits und jenseits des mindestens einen Steifigkeitsänderungselements ausgebildet wird.

Durch das Ausbilden des mindestens einen Steifigkeitsänderungselements können Fertigungstoleranzen beim Herstellen des Verbindungselements beispielsweise am Ende des Fertigungsprozesses oder gegebenenfalls auch noch später, zum Beispiel intra-operativ durch einen Operateur, in gewünschter Weise ausgeglichen werden. Das Ausbilden des mindestens einen Steifigkeitsänderungselements ermöglicht es also, aufgrund von Fertigungstoleranzen bei der Herstellung des Zwischenabschnitts auftretende Abweichungen von der gewünschten Steifigkeit eines Blattfederelements gezielt zu korrigieren.

Besonders einfach herstellen lässt sich das Verbindungselement, wenn das Blattfederelement durch spanende Bearbeitung aus einem Vollmaterial hergestellt wird. Insbesondere können so Oberflächen des Blattfederelements mit hoher Güte ausgebildet werden, welche zu einer Erhöhung der Dauerfestigkeit des Verbindungselements insgesamt beitragen.

Vorteilhaft ist es ferner, wenn der erste und/oder der zweite Befestigungsabschnitt durch spanende Bearbeitung aus einem Vollmaterial hergestellt werden. So kann insbesondere das Verbindungselement insgesamt aus einem Vollmaterial und damit bei Bedarf auch einstückig hergestellt werden.

Das Verbindungselement lässt sich insbesondere mit hoher Qualität und hoher Oberflächengüte auf einfache Weise dadurch herstellen, dass es spanend bearbeitet wird durch Fräsen. Beispielsweise können Ausnehmungen in der oben beschriebenen Weise mit Stirn- oder Schaftfräsern hergestellt werden, Steifigkeitsänderungselemente in Form von Nuten mittels T- oder Schaftfräsern.

Gemäß einer bevorzugten Variante des erfindungsgemäßen Verfahrens kann vorgesehen sein, dass die mindestens eine Ausnehmung durch beidseitiges Fräsen in aufeinander zu weisende Richtungen quer zu einer vom Verbindungselement definierten Längsachse ausgebildet wird und dass eine Frästiefe in einer Richtung quer zur Längsachse insgesamt kleiner ist als eine Dicke des Verbindungselements in Fräsrichtung quer zur Längsachse, so dass ein beide ausgefrästen Vertiefungen voneinander trennendes Trennelement ausgebildet wird. Die Summe der Tiefen beider Vertiefungen ist also kleiner als eine Dicke des Verbindungselements in Fräsrichtung quer zur Längsachse. Das Trennelement kann so insbesondere in der Art einer die Ausnehmungen seitlich verschließenden "Schwimmhaut" ausgebildet werden, welche die ausgefrästen Vertiefungen voneinander trennt. Insbesondere kann das Trennelement über die Windungen des Blattfederelements seitlich vorstehen und so in einer Queransicht eine äußere Kontur des Verbindungselements des Zwischenabschnitts begrenzen.

Besonders einfach ausbilden lässt sich das Trennelement, wenn es wandförmig ausgebildet wird. Beispielsweise kann über eine Wandstärke des Trennelements eine Steifigkeit des Blattfederelements gezielt eingestellt werden.

Insbesondere um eine Steifigkeit des Verbindungselements in gewünschter Weise nachzujustieren, kann das Trennelement teilweise entfernt werden. Beispielsweise kann es durchbrochen oder zur Ausbildung eines streifenförmigen Vorsprungs nahezu vollständig entfernt werden.

Besonders einfach lässt sich das Trennelement teilweise entfernen durch Bohren, Fräsen oder Schneiden. Insbesondere das teilweise Entfernen durch Schneiden lässt sich bei Bedarf auch noch intra-operativ realisieren. Beispielsweise kann das Verbindungselement zunächst mit einer vollständig ausgebildeten Trennwand ausgeliefert werden, so dass diese erst zur besonderen Anpassung an die physiologischen Bedürfnisse eines Patienten in gewünschtem Umfang zum Beispiel durch einen Operateur entfernt werden kann.

Vorteilhafterweise wird das Trennelement quer zur Längsachse teilweise durchbrochen. Auf diese Weise lässt sich eine Steifigkeit des Blattfederelements gezielt herabsetzen.

Die nachfolgende Beschreibung bevorzugter Ausführungsformen der Erfindung dient im Zusammenhang mit der Zeichnung der näheren Erläuterung. Es zeigen:
- Figur 1:: eine schematische Gesamtansicht eines an einer Wirbelsäule festgelegten Wirbelsäulenstabilisierungssystems;
- Figur 2:: eine perspektivische schematische Ansicht eines Verbindungselements des Wirbelsäulenstabilisierungssystems aus Figur 1;
- Figur 3a:: eine schematische Schnittansicht längs Linie 3a-3a in Figur 2;
- Figur 3b:: eine Schnittansicht analog Figur 3a bei einem weiteren Ausführungsbeispiel eines Verbindungselements;
- Figur 4a:: eine perspektivische Ansicht eines weiteren Ausführungsbeispiels eines Verbindungselements;
- Figur 4b:: eine Seitenansicht des Verbindungselements aus Figur 4a;
- Figur 5a:: eine perspektivische Ansicht eines weiteren Ausführungsbeispiels eines Verbindungselements;
- Figur 5b:: eine Seitenansicht des Verbindungselements aus Figur 5a;
- Figur 6a:: eine perspektivische Ansicht eines weiteren Ausführungsbeispiels eines Verbindungselements;
- Figur 6b:: eine Seitenansicht des Verbindungselements aus Figur 6a;
- Figur 7a:: eine perspektivische Ansicht eines weiteren Ausführungsbeispiels eines Verbindungselements;
- Figur 7b:: eine Seitenansicht des Verbindungselements aus Figur 7a;
- Figur 8a:: eine perspektivische Ansicht eines weiteren Ausführungsbeispiels eines Verbindungselements;
- Figur 8b:: eine Seitenansicht des Verbindungselements aus Figur 8a; und
- Figur 9:: eine beispielhafte schematische Darstellung der Steifigkeitszunahme des Zwischenabschnitts in Abhängigkeit der Wandstärke des Blattfederelements, und zwar mit und ohne Steifigkeitsänderungselement in Form eines Vorsprungs.

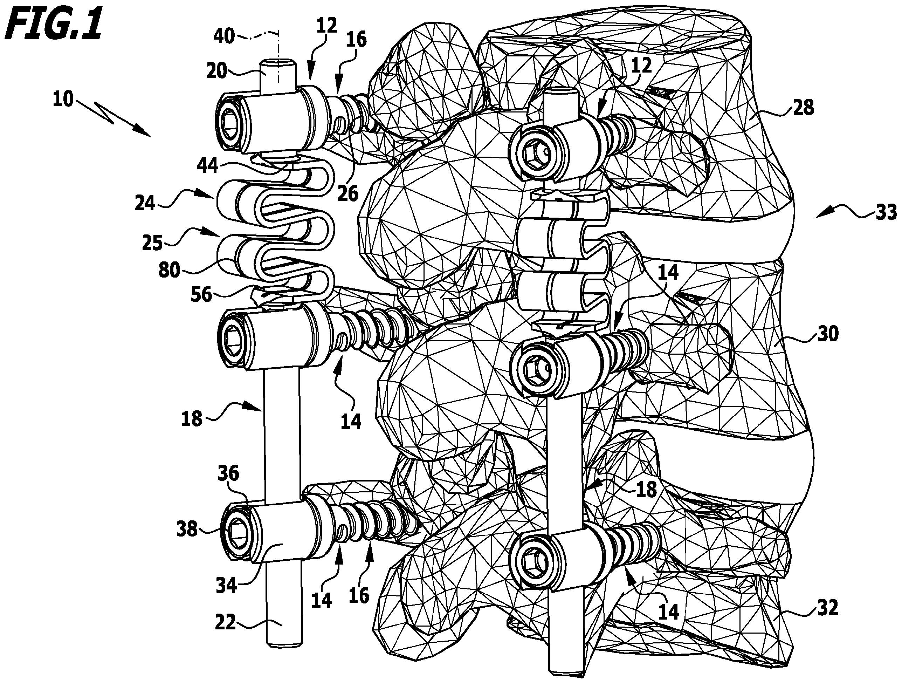

In Figur 1 ist schematisch ein insgesamt mit dem Bezugszeichen 10 bezeichnetes Wirbelsäulenstabilisierungssystem dargestellt. Es umfasst erste Knochenbefestigungseinrichtungen 12 und zweite Knochenbefestigungseinrichtungen 14, die bei dem in Figur 1 schematisch dargestellten Ausführungsbeispiel alle in Form identischer Knochenschrauben 16 ausgebildet sind, jedoch auch unterschiedlich ausgebildet sein können. Ferner umfasst das Wirbelsäulenstabilisierungssystem 10 im Wesentlichen stabförmige Verbindungselemente 18, welche einen ersten Befestigungsabschnitt 20 zum Festlegen an einer Knochenschraube 16, einen zweiten Befestigungsabschnitt 22 zum Festlegen an zwei Knochenschrauben 16 und einen zwischen dem ersten und dem zweiten Befestigungsabschnitt 20, 22 angeordneten oder ausgebildeten, mindestens teilweise flexiblen Zwischenabschnitt 24 in Form eines Blattfederelements 25 umfassen.

Die Knochenschrauben 16 umfassen jeweils einen Knochenverankerungsabschnitt 26 mit einem Knochengewinde zum Verankern in einem Knochen, beispielsweise in einem der in Figur 1 schematisch dargestellten Wirbel 28, 30 und 32 einer Wirbelsäule 33. Des Weiteren umfasst jede Knochenschraube 16 einen in einer Justagestellung gelenkig am Knochenverankerungsabschnitt 26 gelagerten Halteabschnitt 34 in Form eines Gabelkopfes, welcher eine Befestigungsabschnittsaufnahme 36 für einen der Befestigungsabschnitte 20, 22 eines Verbindungselements 18 umfasst. Mittels einer Klemmschraube 38 kann ein in die Befestigungsabschnittsaufnahme 36 eingeführter Befestigungsabschnitt 20, 22 in einer Implantationsstellung klemmend fixiert werden. Durch Festlegen des Befestigungsabschnitts 20, 22 am Halteabschnitt 34 wird vorzugsweise auch eine Relativstellung zwischen dem Knochenverankerungsabschnitt 26 und dem Halteabschnitt 34 dauerhaft festgelegt.

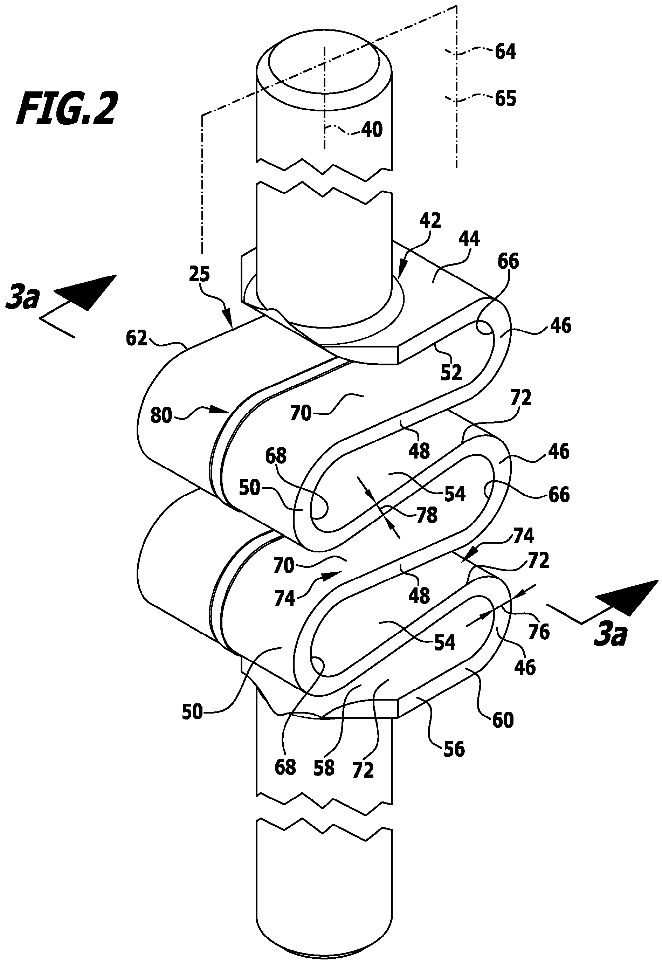

Die Befestigungsabschnitte 20, 22 sind jeweils rundstabförmig ausgebildet und weisen somit einen kreisförmigen Querschnitt auf. Sie sind einstückig mit dem Zwischenabschnitt 24 ausgebildet. Vorzugsweise, wie bei dem in den Figuren 1 und 2 dargestellten Ausführungsbeispiel eines Verbindungselements 18, definieren die Befestigungsabschnitte 20 und 22 eine gemeinsame Längsachse 40, die auch eine Längsachse des Zwischenabschnitts 24 definiert.

Der Zwischenabschnitt 24 ist an ein erstes Ende des Befestigungsabschnitts 20 angeformt und bildet im Übergangsbereich 42 zum Befestigungsabschnitt 20 eine im Wesentlichen flache, quaderförmige Endplatte 44 aus. An einer Längsseite derselben schließt sich quer ab von der Längsachse 40 ein gekrümmter Abschnitt 46 an. Dieser erstreckt sich über einen Winkelbereich von etwas mehr als 180°. An den gekrümmten Abschnitt 46 schließt sich ein flacher ebener Abschnitt 48 an, der wiederum in einen gekrümmten Abschnitt 50 übergeht, welcher ebenfalls von der Längsachse 40 weg weisend konvex gekrümmt ist. Die gekrümmten Abschnitte 46 und 50 weisen jedoch in zueinander entgegengesetzte Richtungen. Eine in Richtung auf den zweiten Befestigungsabschnitt 22 hin weisende Endfläche 52 der Endplatte 44 ist etwas gegen den ebenen Abschnitt 48 geneigt. An den gekrümmten Abschnitt 50 schließt sich wiederum ein ebener Abschnitt 54 an, welcher ebenfalls etwas gegen die Endfläche 52 geneigt verläuft. Die schlangenförmige Kontur des Zwischenabschnitts 24 setzt sich fort mit weiteren gekrümmten Abschnitt 46, einem weiteren, sich daran anschließenden ebenen Abschnitt 48, einem sich daran anschließenden gekrümmten Abschnitt 50, einem sich daran anschließenden ebenen Abschnitt 54 und einem letzten, sich daran anschließenden gekrümmten Abschnitt 46, welcher in eine zur Endplatte 44 entsprechend geformte Endplatte 56 übergeht, welche eine in Richtung des ersten Befestigungsabschnitts 20 weisende Endfläche 58 aufweist, die ebenfalls etwas geneigt relativ zu den ebenen Abschnitten 54, aber parallel zur Endfläche 52 verläuft.

Eine Steifigkeit des Zwischenabschnitts 24 liegt je nach Wahl des Materials, aus welchem das Verbindungselement 18 hergestellt ist, in einem Bereich von etwa 30 N/mm bis etwa 150 N/mm. Der Zwischenabschnitt 24 ist aus einem insgesamt im Wesentlichen flachen blattfederförmigen Material gebildet beziehungsweise aus einem Vollmaterial durch spanende Bearbeitung, beispielsweise Fräsen, oder Erodieren geformt. Quer ab von der Längsachse 40 weist der Zwischenabschnitt 24 zwei in einer Grundstellung, in welcher auf den Zwischenabschnitt 24 keine äußeren Kräfte einwirken, parallel zueinander und voneinander weg weisende Seitenflächen 60 und 62 auf. Diese verlaufen zudem parallel zu einer die Längsachse 40 enthaltenden Symmetrieebene 64 des Verbindungselements 18, welche eine Spiegelebene 54 definiert.

Durch die schlangenförmige Ausgestaltung des Zwischenabschnitts 24 sind bei dem in den Figuren dargestellten Ausführungsbeispiel insgesamt fünf Ausnehmungen 66, 68 ausgebildet, wobei die drei Ausnehmungen 66 in dieselbe Richtung weisen wie die, in welche die konvex gekrümmten Abschnitte 50 weisen. Die zwei Ausnehmungen 68 sind jedoch in die entgegengesetzte Richtung weisend geöffnet, also in die Richtung, in die die konvex gekrümmten Abschnitte 46 weisen. Jede der Ausnehmungen 66, 68 definiert eine Einführöffnung 70 beziehungsweise 72, die jeweils einem gekrümmten Abschnitt 46 beziehungsweise 50 gegenüberliegt und in die jeweils entgegengesetzte Richtung weist. Jede Ausnehmung 66, 68 wird durch zwei in Richtung der jeweiligen Einführöffnung 70, 72 aufeinander zu laufende ebene Abschnitte 48 und 54 begrenzt, so dass sich ein Querschnitt der jeweiligen, in etwa tropfenförmigen Ausnehmung 66, 68 von der Einführöffnung 70 beziehungsweise 72 in Richtung auf den die Ausnehmungen 66, 68 weiter begrenzenden gekrümmten Abschnitte 46, 50 hin zunimmt. Damit definiert jede Einführöffnung 70, 72 eine Engstelle 74. Die Ausnehmungen 66, 68 sind somit jeweils in einer Richtung quer zur Längsachse 40 seitlich geöffnet.

Eine Dicke 76 des Zwischenabschnitts 24 im Bereich der gekrümmten Abschnitte 46, 50 ist größer als im Bereich der ebenen Abschnitte 48, 54. Die Dicke 76 beträgt etwa das 1,1fache bis etwa 1,5fache der Dicke 78, vorzugsweise etwa das 1,3fache bis etwa 1,35fache. Bei dem in den Figuren dargestellten Ausführungsbeispiel beträgt die Dicke 76 etwa 0,8 mm, die Dicke 78 etwa 0,6 mm. Die Dicke 76 liegt vorzugsweise in einem Bereich von etwa 0,5 mm bis etwa 1,5 mm, bevorzugt in einem Bereich von etwa 0,7 mm bis etwa 1,1 mm, weiter bevorzugt in einem Bereich von etwa 0,7 mm bis etwa 0,9 mm. Beispielsweise kann die Dicke 76 einen Wert von etwa 0,975 mm aufweisen. Die Dicke 78 liegt vorzugsweise in einem Bereich von etwa 0,5 mm bis etwa 1,5 mm, bevorzugt in einem Bereich von etwa 0,4 mm bis etwa 0,9 mm, weiter bevorzugt in einem Bereich von etwa 0,5 mm bis etwa 0,7 mm. Beispielsweise kann die Dicke 78 einen Wert von 0,775 mm aufweisen. Ein Innenradius der gekrümmten Abschnitte 46, 50 beträgt bei den in den Figuren dargestellten Ausführungsbeispielen etwa 1,6 mm, ein Außenradius etwa 2,2 mm. Beide Radien können abhängig von der Dicke 76 entsprechend abweichen.

Zur Erhöhung der Dauerfestigkeit des Verbindungselements 18 kann dessen äußere Oberfläche 80 durch ein Strahlverfahren mindestens teilweise bearbeitet sein.

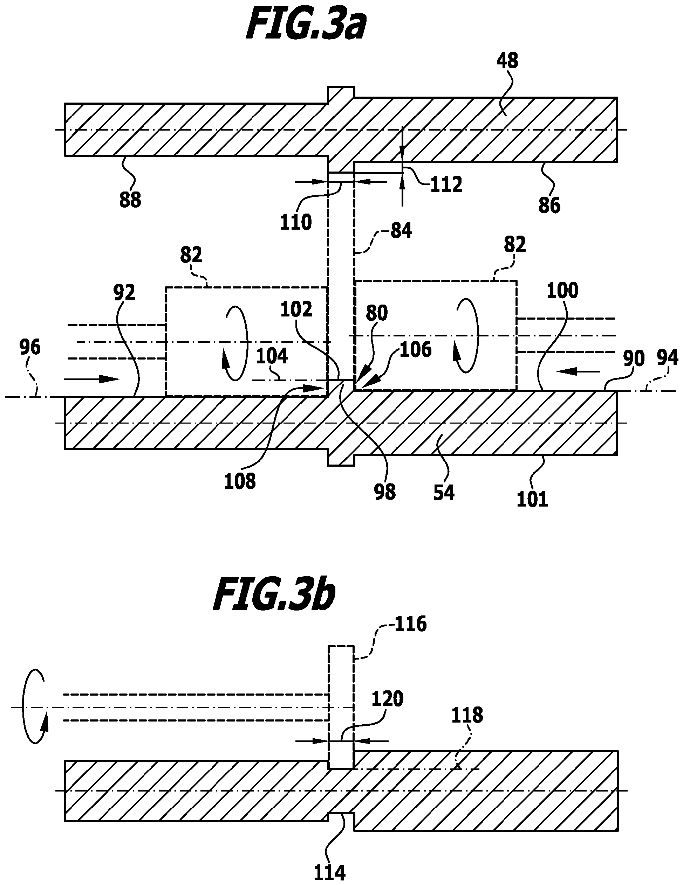

Das Verbindungselement 18 umfasst ferner ein Steifigkeitsänderungselement 80. Dieses kann, wie in den Figuren 3a bis 8b dargestellt, auf unterschiedliche Weise geformt sein und in nachfolgend beschriebener Weise hergestellt werden.

In Figur 3a ist schematisch dargestellt, wie zur Ausbildung der Ausnehmung 68 mittels zweier rotierender Stirn- beziehungsweise Schaftfräser 82, zunächst zwei durch ein Trennelement 84 getrennte Vertiefungen 86 und 88 ausgebildet werden. Aufgrund von Fertigungstoleranzen liegen die die Vertiefungen 86 und 88 begrenzenden Blattfederoberflächenabschnitte 90 und 92 nicht auf derselben Linie, das heißt sie definieren unterschiedliche Niveaus 94 beziehungsweise 96. Würde der die Vertiefung 86 ausbildende Stirn- beziehungsweise Schaftfräser 82 weiter in Richtung auf die Vertiefung 88 hin vorgeschoben werden, wobei dann das Trennelement 84 vollständig entfernt würde, entstünde im Übergangsbereich ein einstufiger Versatz zwischen den Niveaus 94 und 96, welche von den Blattfederoberflächenabschnitten 90 und 92 definiert werden.

Grundsätzlich wäre es möglich, das Trennelement 84, welches wandförmig in der Art einer "Schwimmhaut" ausgebildet ist, zu belassen. Allerdings kann, wie in Figur 3a dargestellt, das Trennelement 84 nahezu vollständig mittels eines der Stirn- beziehungsweise Schaftfräser 82 entfernt werden, wobei es nicht darauf ankommt, ausgehend von welcher Vertiefung 86 oder 88 dies geschieht.

Das Trennelement 84 bei der Herstellung des Verbindungselements 18 zunächst vollständig stehen zu lassen hat insbesondere den Vorteil, dass Fertigungstoleranzen gegenüber einem direkten Durchfräsen weiter verringerbar sind, da eine Grundsteifigkeit des Verbindungselements beim Herstellungsprozess deutlich erhöht ist. Es kann also beim Fräsen des Verbindungselements 18 aus einem Vollmaterial praktisch nicht mehr zu Verwindungen kommen, zumindest lässt sich diese Gefahr deutlich reduzieren.

Wird das Trennelement 84 in der beschriebenen Weise nicht ganz vollständig entfernt, bleibt ein geringer Überstand in Form eines Versteifungsvorsprungs 98, welcher das Steifigkeitsänderungselement 80 definiert. Eine von der Blattfederelementoberfläche 100 abstehende Stirnfläche 102 des Versteifungsvorsprungs 98 definiert ein drittes Niveau 104, so dass insgesamt zwei stufenförmige Absätze 106 und 108 ausgebildet werden, und zwar einerseits zwischen dem Niveau 94 und dem Niveau 102 sowie zwischen dem Niveau 102 und dem Niveau 96.

Eine Breite 110 sowie eine Höhe 112 des Versteifungsvorsprungs 98 bezogen auf eines der beiden Niveaus 94 oder 96 bestimmt letzten Endes eine Steifigkeitsänderung des Zwischenabschnitts 24 im Vergleich zu einer Steifigkeit desselben ohne Steifigkeitsänderungselement 80.

Alternativ zum Versteifungsvorsprung 98 kann, wie schematisch in Figur 3b dargestellt, auch eine Versteifungsnut 114 mittels eines T-Fräsers 116 ausgebildet werden. Bei gleicher Dicke des Blattfederelements 25 kann so eine geringe Steifigkeitsabnahme des Blattfederelements 25 erreicht werden. Vor Ausbildung der Vertiefungsnut 114 wird das vorzugsweise zunächst ausgebildete Trennelement 84 vollständig entfernt, und zwar indem von einer Seite, beispielsweise ausgehend von der Vertiefung 86 mittels des Stirn- oder Schaftfräsers 83, das Trennelement 84 komplett herausgefräst wird. Dadurch entsteht ein direkter Versatz zwischen den Niveaus 94 und 96. Wird im Bereich des so entstandenen Versatzes die Vertiefungsnut 114 eingebracht, wird ein drittes Niveau 118 definiert. Eine Breite 120 der Vertiefungsnut 114 wird im Wesentlichen definiert durch eine Dicke beziehungsweise eine Breite des T-Fräsers 116.

Das Trennelement 84 hat vorzugsweise eine Wandstärke von etwa 0,3 mm bis etwa 0,5 mm. Damit ist es ausreichend stabil, um eine Verformung des Zwischenabschnitts 24 beim spannenden Formen desselben aus einem Vollmaterial zu vermeiden. Andererseits kann ein solches Trennelement 84 durch geringe Schnittkräfte ganz oder teilweise herausgetrennt werden.

In der beschriebenen Weise kann der Blattfederabschnitt 25 sowohl auf der Blattfederelementoberfläche 100 als auch auf einer in entgegensetzter Richtung weisenden Blattfederelementoberfläche 101 mit einem Steifigkeitsänderungselement 80 ausgestattet werden. Denkbar ist es jedoch auch, nur eine der beiden Blattfederelementoberflächen 100 oder 101 mit einem Steifigkeitsänderungselement 80 auszustatten. Selbstverständlich können auch mehrere Steifigkeitsänderungselemente 80 vorgesehen werden, welche wahlweise in Form eines Versteifungsvorsprungs oder einer Versteifungsnut ausgebildet sein können.

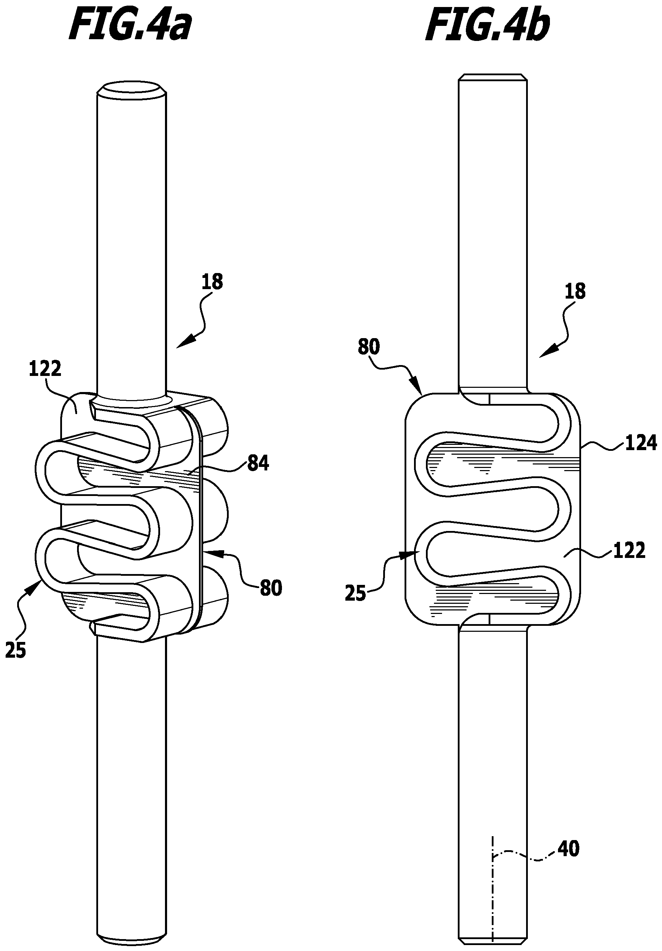

In den Figuren 4a und 4b ist ein weiteres Ausführungsbeispiel eines Verbindungselements 18 dargestellt. Es unterscheidet sich von dem in Figur 2 dargestellten Verbindungselement 18 durch die Ausgestaltung des Steifigkeitsänderungselements 80. Es bildet im Wesentlichen eine durchgehende Trennwand 122, welche das Blattfederelement 25 praktisch vollständig umschließt. In der in Figur 4b dargestellten Seitenansicht des Verbindungselements 18 ist erkennbar, dass eine äußere Kontur 124 der Trennwand 122 in einer Drauf- oder Seitenansicht des Verbindungselements 18 rechteckig mit abgerundeten Ecken geformt ist. In einer Richtung quer zur Längsachse 40 überragt die äußere Kontur 24 das Blattfederelement 25 etwas.

Das in den Figuren 4a und 4b dargestellte Ausführungsbeispiel kann insbesondere auch als Zwischenstufe bei der Herstellung des in Figur 2 dargestellten Ausführungsbeispiels betrachtet werden. Durch ganz oder teilweises Entfernen der Trennwand 122 können wahlweise Versteifungsvorsprünge 98 oder Versteifungsnuten 114 auf den jeweiligen Blattfederelementoberflächen 100 beziehungsweise 101 ausgebildet werden.

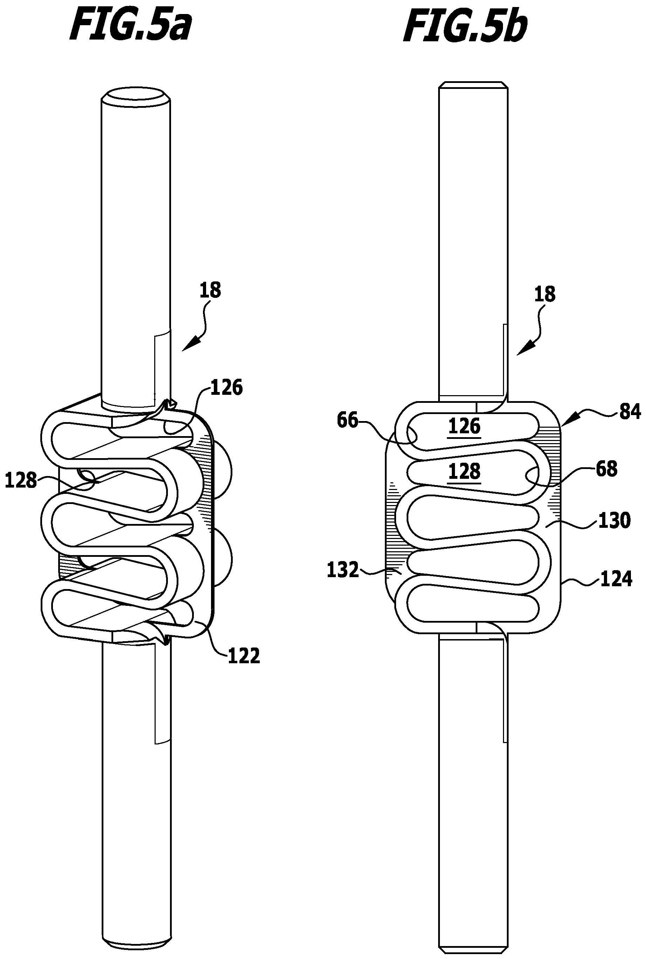

Das in den Figuren 5a und 5b dargestellte Ausführungsbeispiel eines Verbindungselements 18 unterscheidet sich von dem in den Figuren 4a und 4b dargestellten Verbindungselement 18 dadurch, dass im Bereich der Ausnehmungen 66 und 68 Durchbrechungen 126 beziehungsweise 128 vorgesehen sind, welche allseitig umschlossen sind. Die Durchbrechungen 126 und 128 werden jeweils teilweise von der verbleibenden Trennwand 122 und teilweise vom Blattfederelement 25 begrenzt. Insgesamt bleibt so die äußere Kontur 124 der Trennwand 122 nahezu erhalten. Parallel zur Längsachse 40 verbleiben so zwei äußere Stegelemente 130 und 132, welche jeweils benachbarte gekrümmte Abschnitte 46 beziehungsweise benachbarte gekrümmte Abschnitte 50 jeweils paarweise miteinander verbinden und so den Zwischenabschnitt 24 etwas versteifen.

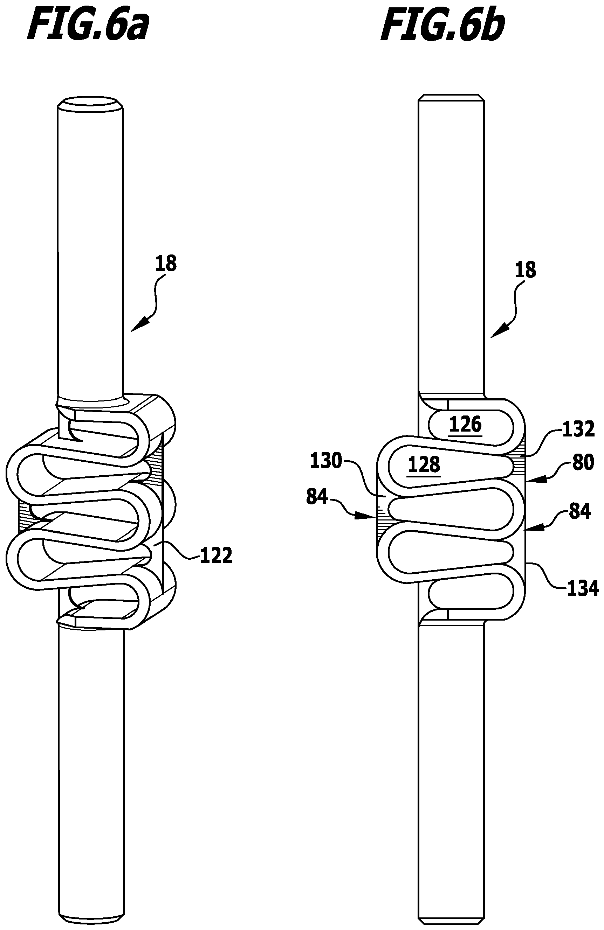

Bei dem in den Figuren 6a und 6b dargestellten weiteren Ausführungsbeispiel eines Verbindungselements 18 ist das Steifigkeitsänderungselement 80 ebenfalls in Form einer teilweise entfernten Trennwand 122 ausgebildet. Dies kann beispielsweise dadurch erreicht werden, dass bei dem in den Figuren 4a und 4b dargestellten Ausführungsbeispiel eines Verbindungselements 18 eine Kontur 134 des Trennelements 84 so gewählt wird, dass sie seitlich bündig mit den gekrümmten Abschnitten 46 beziehungsweise 50 abschließt. Es werden so wiederum Stegelemente 130 und 132 ausgebildet, die benachbarte gekrümmte Abschnitte 46 beziehungsweise 50 jeweils paarweise miteinander verbinden und versteifen. Des Weiteren ist die Trennwand 122 bei dem in den Figuren 6a und 6b dargestellten Verbindungselement 18 ebenfalls mit Durchbrechungen 126 und 128 im Bereich der Ausnehmungen 66 und 68 versehen.

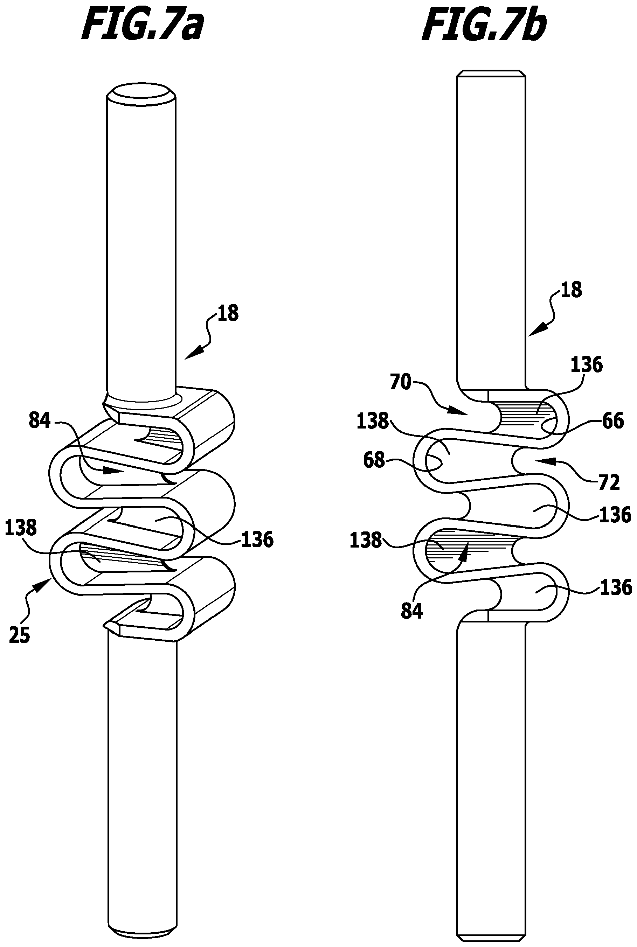

Bei dem in den Figuren 7a und 7b dargestellten Ausführungsbeispiel eines Verbindungselements 18 verbleiben lediglich im Bereich der Ausnehmungen 66 und 68 Trennwände 136 und 138, die jedoch nicht miteinander in Verbindung stehen. Sie erstrecken sich jeweils zwischen einander gegenüberliegenden, aufeinander zu weisenden Oberflächenabschnitten einer der beiden Blattfederelementoberflächen 100 beziehungsweise 101. Die Trennwände 136 und 138 erstrecken sich nicht ganz bis in den Bereich der Einführöffnungen 70 und 72, schließen sich jedoch direkt an die Ausnehmungen 66 und 68 begrenzende Innenflächen der gekrümmten Abschnitte 46 und 50 an.

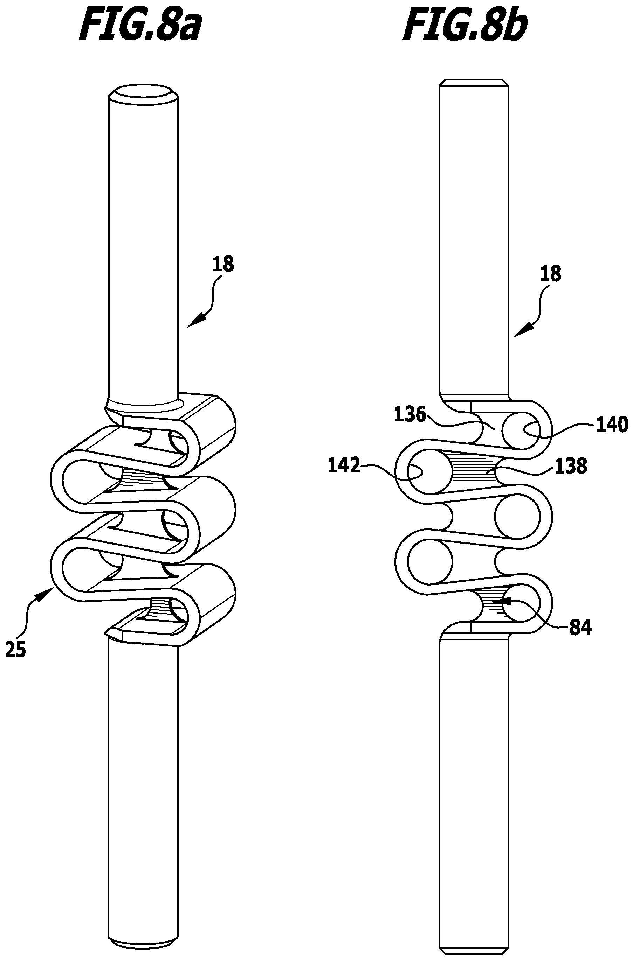

Das in den Figuren 8a und 8b dargestellte Ausführungsbeispiel unterscheidet sich vom Verbindungselement 18, wie es in den Figuren 7a und 7b dargestellt ist, dadurch, dass es, in der Seitenansicht in Figur 8b erkennbare, kreisförmige Durchbrechungen der Trennwände 136 und 138 direkt angrenzend an die gekrümmten Abschnitte 46 und 50 im Bereich der Ausnehmungen 66 und 68 aufweist. Dadurch entstehen stegförmige Trennwandabschnitte, welche einander gegenüberliegende ebene Abschnitte 48 beziehungsweise 54 miteinander verbinden und die Steifigkeit des Blattfederelements 25 auf diese Weise etwas erhöhen.

Die in den Figuren 4a bis 8b dargestellten Ausführungsbeispiele von Verbindungselementen 18 können, wie in Verbindung mit den Figuren 3a und 3b beschrieben, lediglich Vorstufen zur Ausbildung von Versteifungsvorsprüngen und Versteifungsnuten bilden, welche beispielsweise nur in dem Bereich ausgebildet werden, in welchem die Verbindungselemente 18, wie sie in den Figuren 4a bis 8b dargestellt und beschrieben Trennelemente 84 aufweisen.

Die Trennelemente 84, die schwimmhautartig ausgebildet sind, können auch zur Feineinstellung der Federsteifigkeit in Richtung der Längsachse 40 verwendet werden. Beim Herstellungsprozess lassen sich tatsächlich erreichte Nennmaße innerhalb der Toleranz messen. In einem nächsten Schritt wird das notwendige Maß beziehungsweise der Überstand für das dann noch verbleibende Trennelement 84 bestimmt und entsprechend gefräst. Insbesondere können so die Breite 110 sowie die Höhe 112 eines Verstreifungsvorsprungs 98, in analoger Weise auch die Abmessungen einer Versteifungsnut, sowie gegebenenfalls die Größe der Durchbrechungen 126 und 128 beziehungsweise 140 und 142 bestimmt werden. Dies ist dadurch möglich, dass der geometrische Einfluss des verbleibenden Trennelements 84 in Form beispielsweise des Verstreifungsvorsprungs 98 wesentlich geringer ist als die Blattfederdicke selbst.

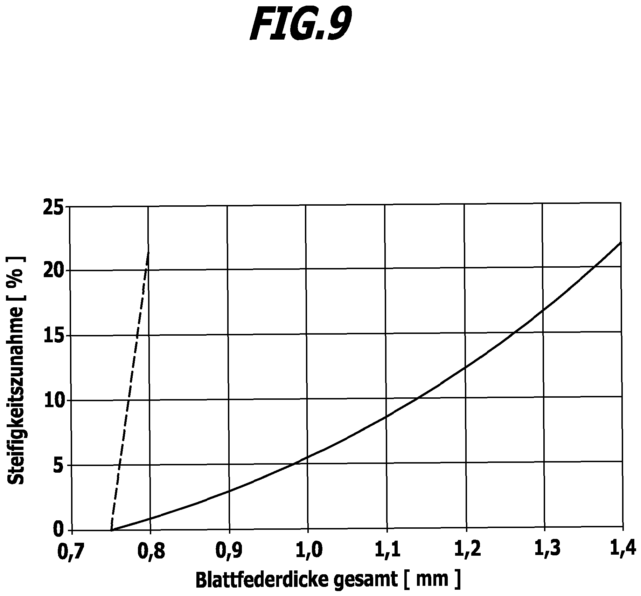

Figur 9 zeigt ein Diagramm, in welchem der Einfluss der Dicke des Blattfederelements 25 ohne Versteifungsvorsprung 98 (gestrichelt gezeichnet) beziehungsweise der Einfluss der Gesamtdicke des Blattfederelements 25 mit Versteifungsvorsprung 98 (durchgezogen gezeichnet) auf eine Zunahme der Steifigkeit dargestellt ist. Bei dem angenommenen Beispiel erkennt man, dass die axiale Steifigkeit durch eine Änderung beispielsweise einer Dicke 76 beziehungsweise 78 von 0,75 mm auf 0,8 mm um 20 % zunimmt, wenn kein Verstreifungsvorsprung vorgesehen ist. Im Gegensatz dazu erreicht man die gleiche Zunahme der Steifigkeit erst mit einer wesentlich größeren Zunahme des Verstreifungsvorsprungs 98, hier beispielsweise etwa 0,3 mm pro Seite.

Das Diagramm in Figur 9 zeigt somit anschaulich, dass Steifigkeitsänderungen, die durch Toleranzen der Dicke 76 beziehungsweise 78 hervorgerufen werden, sehr empfindlich reagieren. Allerdings können sie durch weitaus weniger empfindliche Toleranzen der Höhe des Versteifungsvorsprungs 98 oder gegebenenfalls einer entsprechenden Versteifungsnut kompensiert werden.

Wie bereits oben beschrieben, besteht auch die Möglichkeit, das Trennelement 84, wie beispielsweise in den Figuren 4a und 4b dargestellt, stehen zu lassen.

Dies ermöglicht es einem Operateur intra-operativ nach Bedarf einen Teil des Trennelements 84 herauszutrennen. Dadurch besteht die Möglichkeit, die Steifigkeit des Verbindungselements 18 individuell einzustellen. Das Heraustrennen kann zum Beispiel mit einer Stanze erfolgen, die zum Blattfederelement 25 korrespondierende Maulteilgeometrien aufweist.

Die oben beschriebenen Blattfederelemente 25 weisen vorzugsweise zwei Absätze auf, welche es ermöglichen, dass beim Fertigungsprozess eine höhere Stabilität des Blattfederelements 25 erreicht wird. Auf diese Weise lassen sich insbesondere Fertigungstoleranzen ausgleichen und die Oberflächenqualität des Zwischenabschnitts 24 verbessern. Wie dargelegt können ein Versteifungsvorsprung 98 oder eine Versteifungsnut 114 zur Feinjustierung der axialen Steifigkeit verwendet werden, sowohl im Fertigungsprozess als auch intra-operativ.

Die beschriebenen Verbindungselemente 18 können aus metallischen Materialien oder aus Kunststoffen hergestellt sein, beispielsweise aus den bereits oben genannten Materialien.

## Patentansprüche

1. Verbindungselement (18) für ein Wirbelsäulenstabilisierungssystem (10) mit einem ersten Befestigungsabschnitt (20) zum Festlegen an einer ersten Knochenbefestigungseinrichtung (12), einem zweiten Befestigungsabschnitt (22) zum Festlegen an einer zweiten Knochenbefestigungseinrichtung (14) und einem zwischen dem ersten und dem zweiten Befestigungsabschnitt (20, 22) angeordneten oder ausgebildeten, mindestens teilweise flexiblen Zwischenabschnitt (24), welcher Zwischenabschnitt (24) in Form eines streifenförmigen, gewundenen Blattfederelements (25) ausgebildet ist und mindestens eine in einer Richtung quer zu einer vom Zwischenabschnitt (24) definierten Längsachse (40) seitlich geöffnete Ausnehmung (66, 68) aufweist, wobei mindestens eine der beiden Blattfederelementoberflächen (100, 101) des Blattfederelements (25) mindestens ein Steifigkeitsänderungselement (80) umfasst und sich das mindestens eine Steifigkeitsänderungselement (80) in einer vom Blattfederelement (25) definierten Längsrichtung erstreckt, **dadurch gekennzeichnet, dass** das Blattfederelement (25) unterschiedliche Dicken diesseits und jenseits des mindestens einen Steifigkeitsänderungselements (80) aufweist, bezogen auf die vom Blattfederelement definierte Längsrichtung.

2. Verbindungselement nach Anspruch 1, **dadurch gekennzeichnet, dass** das mindestens eine Steifigkeitsänderungselement (80) streifenförmig ausgebildet ist.

3. Verbindungselement nach einem der voranstehenden Ansprüche, **dadurch gekennzeichnet, dass** beide Blattfederelementoberflächen (100, 101) des Blattfederelements (25) jeweils mindestens ein Steifigkeitsänderungselement (80) umfassen.

4. Verbindungselement nach einem der voranstehenden Ansprüche, **dadurch gekennzeichnet, dass** sich das mindestens eine Steifigkeitsänderungselement (80) zwischen zwei aufeinander zu weisenden Oberflächenabschnitten (90, 92) einer der beiden Blattfederelementoberflächen (100, 101) erstreckt.

5. Verbindungselement nach einem der voranstehenden Ansprüche, **dadurch gekennzeichnet, dass** das mindestens eine Steifigkeitsänderungselement (80) im Bereich der mindestens einen Ausnehmung (66, 68) angeordnet ist.

6. Verbindungselement nach einem der voranstehenden Ansprüche, **dadurch gekennzeichnet, dass** das mindestens eine Steifigkeitsänderungselement (80) in Form eines von einer der beiden Blattfederelementoberflächen (100, 101) abstehenden Versteifungsvorsprungs (98) oder in Form einer sich von einer der beiden Blattfederelementoberflächen (100, 101) in das Blattfederelement (25) hinein erstreckenden Versteifungsnut (114) ausgebildet ist.

7. Verbindungselement nach einem der voranstehenden Ansprüche, **dadurch gekennzeichnet, dass** eine Dicke (110; 120) des mindestens einen Steifigkeitsänderungselements (80) in einer Richtung quer oder im Wesentlichen quer zu einer vom Verbindungselement (18) definierten Längsachse (40) maximal etwa einer Dicke (76, 78) des Blattfederelements (25) entspricht und dass die Dicke (110; 120) des mindestens einen Steifigkeitsänderungselements (80) in einem Bereich von etwa 0,2 mm bis etwa 0,6 mm liegt, vorzugsweise in einem Bereich von etwa 0,3 mm bis etwa 0,5 mm.

8. Verbindungselement nach einem der voranstehenden Ansprüche **dadurch gekennzeichnet, dass** eine Dicke (76, 78) des Blattfederelements (25) in einem Bereich von etwa 0,5 mm bis etwa 0,9 mm liegt, vorzugsweise in einem Bereich von etwa 0,6 mm bis etwa 0,8 mm.

9. Verbindungselement nach einem der voranstehenden Ansprüche, **dadurch gekennzeichnet, dass** mindestens eine der beiden Blattfederelementoberflächen (100, 101) mindestens zwei stufenförmig ausgebildete Absätze (106, 108) aufweist, so dass die mindestens eine der beiden Blattfederelementoberflächen (100, 101) mindestens zwei, vorzugsweise drei ebene, parallel zueinander verlaufende Blattfederoberflächenabschnitte (94, 96, 104; 94, 96, 118) aufweist.

10. Wirbelsäulenstabilisierungssystem (10) umfassend mindestens eine erste Knochenbefestigungseinrichtung (12), mindestens eine zweite Knochenbefestigungseinrichtung (14) und ein Verbindungselement (18), welches Verbindungselement (18) einen ersten Befestigungsabschnitt (20) zum Festlegen an der mindestens einen ersten Knochenbefestigungseinrichtung (12), einen zweiten Befestigungsabschnitt (22) zum Festlegen an der mindestens einen zweiten Knochenbefestigungseinrichtung (14) und einen zwischen dem ersten und dem zweiten Befestigungsabschnitt (20, 22) angeordneten oder ausgebildeten, mindestens teilweise flexiblen Zwischenabschnitt (24) umfasst, welcher Zwischenabschnitt (24) in Form eines streifenförmigen, gewundenen Blattfederelements (25) ausgebildet ist und mindestens eine in einer Richtung quer zu einer vom Zwischenabschnitt (24) definierten Längsachse (40) seitlich geöffnete Ausnehmung (66, 68) aufweist, wobei mindestens eine der beiden Blattfederelementoberflächen (100, 101) des Blattfederelements (25) mindestens ein Steifigkeitsänderungselement (80) umfasst und sich das mindestens eine Steifigkeitsänderungselement (80) in einer vom Blattfederelement (25) definierten Längsrichtung erstreckt, **dadurch gekennzeichnet, dass** das Blattfederelement (25) unterschiedliche Dicken diesseits und jenseits des mindestens einen Steifigkeitsänderungselements (80) aufweist, bezogen auf die vom Blattfederelement definierte Längsrichtung.

11. Wirbelsäulenstabilisierungssystem nach Anspruch 10, **gekennzeichnet durch** ein Verbindungselement (18) nach einem der Ansprüche 2 bis 9.

12. Verfahren zum Herstellen eines Verbindungselements (18) für ein Wirbelsäulenstabilisierungssystem (10) mit einem ersten Befestigungsabschnitt (20) zum Festlegen an einer ersten Knochenbefestigungseinrichtung (12), einem zweiten Befestigungsabschnitt (22) zum Festlegen an einer zweiten Knochenbefestigungseinrichtung (14) und einem zwischen dem ersten und dem zweiten Befestigungsabschnitt (20, 22) angeordneten oder ausgebildeten, mindestens teilweise flexiblen Zwischenabschnitt (24), welcher Zwischenabschnitt (24) in Form eines streifenförmigen, gewundenen Blattfederelements (25) ausgebildet ist und mindestens eine in einer Richtung quer zu einer vom Zwischenabschnitt (24) definierten Längsachse (40) seitlich geöffnete Ausnehmung (66, 68) aufweist, wobei auf mindestens einer der beiden Blattfederelementoberflächen (100, 101) des Blattfederelements (25) mindestens ein Steifigkeitsänderungselement (80) ausgebildet wird und wobei mindestens ein Steifigkeitsänderungselement (80) in einer vom Blattfederelement (25) definierten Längsrichtung erstreckend ausgebildet wird, **dadurch gekennzeichnet, dass** das Blattfederelement (25) mit unterschiedlichen Dicken diesseits und jenseits des mindestens einen Steifigkeitsänderungselements (80) ausgebildet wird, bezogen auf die vom Blattfederelement definierte Längsrichtung.

13. Verfahren nach Anspruch 12, **dadurch gekennzeichnet, dass** die mindestens eine Ausnehmung (66, 68) durch beidseitiges Fräsen in aufeinander zu weisende Richtungen quer zu einer vom Verbindungselement (18) definierten Längsachse (40) ausgebildet wird und dass eine Frästiefe in einer Richtung quer zur Längsachse (40) insgesamt kleiner ist als eine Dicke des Verbindungselements (18) in Fräsrichtung quer zur Längsachse (40), so dass ein beide ausgefrästen Vertiefungen (86, 88) voneinander trennendes Trennelement (84) ausgebildet wird.

14. Verfahren nach Anspruch 13, **dadurch gekennzeichnet, dass** das Trennelement (84) wandförmig ausgebildet wird.

15. Verfahren nach Anspruch 13 oder 14, **dadurch gekennzeichnet, dass** das Trennelement (84) teilweise entfernt wird.

## Claims

1. Connecting element (18) for a spinal column stabilization system (10) comprising a first attachment section (20) for fixing to a first bone attachment device (12), a second attachment section (22) for fixing to a second bone attachment device (14) and an at least partially flexible intermediate section (24) which is arranged or formed between the first and the second attachment section (20, 22), which intermediate section (24) is in the form of a strip-like, winding leaf spring element (25) and comprises at least one recess (66, 68) which is open to the side in a direction transverse to a longitudinal axis (40) defined by the intermediate section (24), wherein at least one of the two leaf spring element surfaces (100, 101) of the leaf spring element comprises at least one stiffness-modifying element (80) and the at least one stiffness-modifiying element (80) extends in a longitudinal direction defined by the leaf spring element (25), **characterized in that** the leaf spring element (25) is of a different thickness on the one side and on the other side of the at least one stiffness-modifying element (80) according to the longitudinal direction defined by the leaf spring element.

2. Connecting element in accordance with claim 1, **characterized in that** the at least one stiffness-modifying element (80) is formed in strip-like manner.

3. Connecting element in accordance with any of the preceding claims, **characterized in that** both leaf spring element surfaces (100, 101) of the leaf spring element (25) each comprise at least one stiffness-modifying element (80).

4. Connecting element in accordance with any of the preceding claims, **characterized in that** the at least one stiffness-modifying element (80) extends between two mutually facing surface sections (90, 92) of one of the two surfaces (100, 101) of the leaf spring element.

5. Connecting element in accordance with any of the preceding claims, **characterized in that** the at least one stiffness-modifying element (80) is arranged in the region of the at least one recess (66, 68).

6. Connecting element in accordance with any of the preceding claims, **characterized in that** the at least one stiffness-modifying element (80) is in the form of a stiffening projection (98) which protrudes from one of the two leaf spring element surfaces (100, 101) or is in the form of a stiffening groove (114) which extends from one of the two surfaces (100, 101) into the leaf spring element (25).

7. Connecting element in accordance with any of the preceding claims, **characterized in that** a thickness (110; 120) of the at least one stiffness-modifying element (80) in a direction transverse or substantially transverse to a longitudinal axis (40) defined by the connecting element (18) corresponds maximally to approximately a thickness (76, 78) of the leaf spring element (25) and **in that** the thickness (110; 120) of the at least one stiffness-modifying element (80) lies in a range extending from approximately 0.2 mm up to approximately 0.6 mm, preferably in a range extending from approximately 0.3 mm up to approximately 0.5 mm.

8. Connecting element in accordance with any of the preceding claims **characterized in that** a thickness (76, 78) of the leaf spring element (25) lies in a range from approximately 0.5 mm up to approximately 0.9 mm, preferably within a range from approximately 0.6 mm up to approximately 0.8 mm.

9. Connecting element in accordance with any of the preceding claims, **characterized in that** at least one of the two leaf spring element surfaces (100, 101) comprises at least two step-like shoulders (106, 108) so that the at least one of the two surfaces (100, 101) of the leaf spring element comprises at least two, preferably three flat, mutually parallel leaf spring surface sections (94, 96, 104; 94, 96, 118).

10. Spinal column stabilization system (10) comprising at least one first bone attachment device (12), at least one second bone attachment device (14) and a connecting element (18), which connecting element (18) comprises a first attachment section (20) for fixing to the at least one first bone attachment device (12), a second bone attachment section (22) for fixing to the at least one second bone attachment device (14) and an at least partially flexible intermediate section (24) that is arranged or formed between the first and the second attachment section (20, 22), which intermediate section (24) is in the form of a strip-like, winding leaf spring element (25) and comprises at least one recess (66, 68) which is open to the side in a direction transverse to a longitudinal axis (40) defined by the intermediate section (24), wherein at least one of the two leaf spring element surfaces (100, 101) of the leaf spring element (25) comprises at least one stiffness-modifying element (80) and the at least one stiffness-modifiying element (80) extends in a longitudinal direction defined by the leaf spring element (25), **characterized in that** the leaf spring element (25) is of a different thickness on the one side and on the other side of the at least one stiffness-modifying element (80) according to the longitudinal direction defined by the leaf spring element.

11. Spinal column stabilization system in accordance with claim 10, **characterized by** a connecting element (18) in accordance with any of the claims 2 to 9.

12. Method of manufacturing a connecting element (18) for a spinal column stabilization system (10) comprising a first attachment section (20) for fixing to a first bone attachment device (12), a second bone attachment section (22) for fixing to a second bone attachment device (14) and an at least partially flexible intermediate section (24) that is arranged or formed between the first and the second attachment section (20, 22), which intermediate section (24) is in the form of a strip-like, winding leaf spring element (25) and comprises at least one recess (66, 68) which is open to the side in a direction transverse to a longitudinal axis (40) defined by the intermediate section (24), wherein at least one stiffness-modifying element (80) is formed on at least one of the two leaf spring element surfaces (100, 101) of the leaf spring element (25) and the at least one stiffness-modifiying element (80) is formed to extend in a longitudinal direction defined by the leaf spring element (25), **characterized in that** the leaf spring element (25) is of a different thickness on the one side and on the other side of the at least one stiffness-modifying element (80) according to the longitudinal direction defined by the leaf spring element.

13. Method in accordance with claim 12, **characterized in that** the at least one recess (66, 68) is formed by milling from both sides in mutually facing directions that are transverse to a longitudinal axis (40) defined by the connecting element (18) and **in that** an overall milling depth is smaller in a direction transverse to the longitudinal axis (40) than a thickness of the connecting element (18) in a milling direction transverse to the longitudinal axis (40), so that a partitioning element (84) separating the two milled-out indentations (86, 88) from each other is formed.

14. Method in accordance with claim 13, **characterized in that** the partitioning element (84) is in the form of a wall.

15. Method in accordance with claim 13 or 14, **characterized in that** the partitioning element (84) is partly removed.

## Revendications

1. Elément de liaison (18) pour un système de stabilisation de colonne vertébrale (10), comprenant un premier tronçon de fixation (20) destiné à être fixé à un premier dispositif de fixation à l'os (12), un deuxième tronçon de fixation (22) destiné à être fixé à un deuxième dispositif de fixation à l'os (14), et un tronçon intermédiaire (24) au moins partiellement flexible, agencé ou formé entre le premier et le deuxième tronçon de fixation (20, 22), ledit tronçon intermédiaire (24) étant réalisé sous la forme d'un élément de ressort à lame (25) sinueux et en forme de bande, et présentant au moins un évidement (66, 68) ouvert latéralement dans une direction transversale à un axe longitudinal (40) défini par le tronçon intermédiaire (24), élément de liaison
dans lequel au moins l'une des deux surfaces d'élément de ressort à lame (100, 101) de l'élément de ressort à lame (25) comporte au moins un élément de variation de raideur (80), et ledit au moins un élément de variation de raideur (80) s'étend dans une direction longitudinale définie par l'élément de ressort à lame (25),
**caractérisé en ce que** l'élément de ressort à lame (25) présente des épaisseurs différentes de part et d'autre dudit au moins un élément de variation de raideur (80) en se référant à la direction longitudinale définie par l'élément de ressort à lame.

2. Elément de liaison selon la revendication 1,
**caractérisé en ce que** ledit au moins un élément de variation de raideur (80) est d'une configuration en forme de bande.

3. Elément de liaison selon l'une des revendications précédentes, **caractérisé en ce que** les deux surfaces d'élément de ressort à lame (100, 101) de l'élément de ressort à lame (25) comportent chacune au moins un élément de variation de raideur (80).

4. Elément de liaison selon l'une des revendications précédentes, **caractérisé en ce que** ledit au moins un élément de variation de raideur (80) s'étend entre deux secteurs de surface (90, 92), dirigés l'un vers l'autre, de l'une des deux surfaces d'élément de ressort à lame (100, 101).

5. Elément de liaison selon l'une des revendications précédentes, **caractérisé en ce que** ledit au moins un élément de variation de raideur (80) est agencé dans la zone dudit au moins un évidement (66, 68).

6. Elément de liaison selon l'une des revendications précédentes, **caractérisé en ce que** ledit au moins un élément de variation de raideur (80) est réalisé sous la forme d'une protubérance de stabilisation (98) faisant saillie de l'une des deux surfaces d'élément de ressort à lame (100, 101), ou bien sous la forme d'une rainure de stabilisation (114) s'étendant vers l'intérieur de l'élément de ressort à lame (25) à partir de l'une des deux surfaces d'élément de ressort à lame (100, 101).

7. Elément de liaison selon l'une des revendications précédentes, **caractérisé en ce qu'**une épaisseur (110; 120) dudit au moins un élément de variation de raideur (80) dans une direction transversale ou sensiblement transversale à un axe longitudinal (40) défini par l'élément de liaison (18), correspond au maximum environ à une épaisseur (76, 78) de l'élément de ressort à lame (25), et **en ce que** l'épaisseur (110; 120) dudit au moins un élément de variation de raideur (80) se situe dans une plage d'environ 0,2 mm à environ 0,6 mm, de préférence dans une plage d'environ 0,3 mm à environ 0,5 mm.

8. Elément de liaison selon l'une des revendications précédentes, **caractérisé en ce qu'**une épaisseur (76, 78) de l'élément de ressort à lame (25) se situe dans une plage d'environ 0,5 mm à environ 0,9 mm, de préférence dans une plage d'environ 0,6 mm à environ 0,8 mm.

9. Elément de liaison selon l'une des revendications précédentes, **caractérisé en ce que** l'une au moins des deux surfaces d'élément de ressort à lame (100, 101) présente au moins deux décrochements (106, 108) en forme de gradin, de sorte qu'au moins l'une des deux surfaces d'élément de ressort à lame (100, 101) présente au moins deux, de préférence trois secteurs de surface de ressort à lame (94, 96, 104; 94, 96, 118) plans s'étendant parallèlement les uns aux autres.

10. Système de stabilisation de colonne vertébrale (10) comprenant au moins un premier dispositif de fixation à l'os (12), au moins un deuxième dispositif de fixation à l'os (14) et un élément de liaison (18), ledit élément de liaison (18) comprenant un premier tronçon de fixation (20) destiné à être fixé audit au moins un premier dispositif de fixation à l'os (12), un deuxième tronçon de fixation (22) destiné à être fixé au dit au moins un deuxième dispositif de fixation à l'os (14), et un tronçon intermédiaire (24) au moins partiellement flexible, agencé ou formé entre le premier et le deuxième tronçon de fixation (20, 22), ledit tronçon intermédiaire (24) étant réalisé sous la forme d'un élément de ressort à lame (25) sinueux et en forme de bande, et présentant au moins un évidement (66, 68) ouvert latéralement dans une direction transversale à un axe longitudinal (40) défini par le tronçon intermédiaire (24), système
dans lequel au moins l'une des deux surfaces d'élément de ressort à lame (100, 101) de l'élément de ressort à lame (25) comporte au moins un élément de variation de raideur (80), et ledit au moins un élément de variation de raideur (80) s'étend dans une direction longitudinale définie par l'élément de ressort à lame (25),
**caractérisé en ce que** l'élément de ressort à lame (25) présente des épaisseurs différentes de part et d'autre dudit au moins un élément de variation de raideur (80) en se référant à la direction longitudinale définie par l'élément de ressort à lame.

11. Système de stabilisation de colonne vertébrale selon la revendication 10, **caractérisé par** un élément de liaison (18) selon l'une des revendications 2 à 9.

12. Procédé de fabrication d'un élément de liaison (18) pour un système de stabilisation de colonne vertébrale (10) comprenant un premier tronçon de fixation (20) destiné à être fixé à un premier dispositif de fixation à l'os (12), un deuxième tronçon de fixation (22) destiné à être fixé à un deuxième dispositif de fixation à l'os (14), et un tronçon intermédiaire (24) au moins partiellement flexible, agencé ou formé entre le premier et le deuxième tronçon de fixation (20, 22), ledit tronçon intermédiaire (24) étant réalisé sous la forme d'un élément de ressort à lame (25) sinueux et en forme de bande, et présentant au moins un évidement (66, 68) ouvert latéralement dans une direction transversale à un axe longitudinal (40) défini par le tronçon intermédiaire (24), procédé
d'après lequel on réalise sur au moins l'une des deux surfaces d'élément de ressort à lame (100, 101) de l'élément de ressort à lame (25), au moins un élément de variation de raideur (80), et ledit au moins un élément de variation de raideur (80) est réalisé de manière s'étendre dans une direction longitudinale définie par l'élément de ressort à lame (25),
**caractérisé en ce que** l'élément de ressort à lame (25) est réalisé avec des épaisseurs différentes de part et d'autre dudit au moins un élément de variation de raideur (80) en se référant à la direction longitudinale définie par l'élément de ressort à lame.

13. Procédé selon la revendication 12, **caractérisé en ce que** ledit au moins un évidement (66, 68) est réalisé par fraisage à partir de deux côtés, dans des directions orientées l'une vers l'autre, transversalement à un axe longitudinal (40) défini par l'élément de liaison (18), et **en ce qu'**une profondeur de fraisage dans une direction transversale à l'axe longitudinal (40) est globalement inférieure à une épaisseur de l'élément de liaison (18) dans la direction de fraisage transversalement à l'axe longitudinal (40), de sorte qu'il se forme ainsi un élément de séparation (84), qui sépare l'un de l'autre les deux creux (86, 88) obtenus par le fraisage.

14. Procédé selon la revendication 13, **caractérisé en ce que** l'élément de séparation (84) est d'une configuration en forme de paroi.

15. Procédé selon la revendication 13 ou la revendication 14, **caractérisé en ce que** l'on élimine au moins partiellement l'élément de séparation (84).
